# EUROPÄISCHE PATENTSCHRIFT

(11) **EP 3 368 503 B1**
(45) Veröffentlichungstag und Bekanntmachung des Hinweises auf die Patenterteilung: **04.11.2020**
(21) Anmeldenummer: 16766929.0
(22) Anmeldetag: 14.09.2016
(51) Int. Cl.: C07C 69/738, C11B 9/00, C11D 3/20, C11D 3/50, A61K 8/37, A61L 2/00, A61Q 13/00, A61Q 19/00

(54) **PHOTOLABILE DUFTSPEICHERSTOFFE**
PHOTOLABILE PRO-FRAGRANCES
INGRÉDIENTS ODORIFÉRANTS PHOTOLABILES

(30) Priorität: 27.10.2015 DE 102015220928
(43) Veröffentlichungstag der Anmeldung: 05.09.2018
(73) Patentinhaber: Henkel AG & Co. KGaA, 40589 Düsseldorf (DE)
(72) Erfinder: KROPF, Christian, 40724 Hilden (DE); GERKE, Thomas, 40627 Düsseldorf (DE); HUCHEL, Ursula, 50733 Köln (DE); GRIESBECK, Axel, 50935 Köln (DE); LANDES, Agnieszka, 33378 Rheda-Wiedenbrück (DE)
(86) Internationale Anmeldenummer: PCT/EP2016/071689
(87) Internationale Veröffentlichungsnummer: WO 2017/071873

(56) Entgegenhaltungen:
- WO-A1-2015/124671
- DANIELE DONDI ET AL: "Interactions between different solar UVB/UVA filters contained in commercial suncreams and consequent loss of UV protection", PHOTOCHEMICAL AND PHOTOBIOLOGICAL SCIENCES, Bd. 5, Nr. 9, 3. August 2006 (2006-08-03), Seite 835, XP055317256, GB ISSN: 1474-905X, DOI: 10.1039/b606768a

## Beschreibung

Die vorliegende Erfindung liegt auf dem Gebiet der Duftspeicherstoffe, wie sie zum Beispiel auf dem Gebiet der Wasch- oder Reinigungsmittel, kosmetischen Mittel sowie Luftpflegemittel Einsatz finden. Die Erfindung betrifft insbesondere spezielle Ketone, die als photolabile Duftspeicherstoffe fungieren. Ferner betrifft die vorliegende Erfindung Wasch- oder Reinigungsmittel, kosmetische Mittel sowie Luftpflegemittel, welche solche Duftspeicherstoffe enthalten. Ferner betrifft sie ein Verfahren zur lang anhaltenden Beduftung von Oberflächen und ebenso ein Verfahren zur lang anhaltenden Raumbeduftung.

Wasch- oder Reinigungsmittel bzw. kosmetische Mittel enthalten zumeist Duftstoffe, die den Mitteln einen angenehmen Geruch verleihen. Die Duftstoffe maskieren dabei zumeist den Geruch der anderen Inhaltstoffe, so dass beim Verbraucher ein angenehmer Geruchseindruck entsteht.

Insbesondere im Bereich Waschmittel sind Duftstoffe wichtige Bestandteile der Zusammensetzung, da die Wäsche sowohl im feuchten als auch im trockenen Zustand einen angenehmen und möglichst auch frischen Duft aufweisen soll. Die Verwendung von Duftstoffen ist grundsätzlich problematisch, da es sich bei diesen um mehr oder minder leicht flüchtige Verbindungen handelt, aber dennoch ein lange anhaltender Dufteffekt angestrebt wird. Insbesondere bei denjenigen Riechstoffen, die die frischen und leichten Noten des Parfüms darstellen und infolge ihres hohen Dampfdrucks besonders flüchtig sind, ist die gewünschte Langlebigkeit des Dufteindrucks kaum erreichbar.

Eine verzögerte Duftfreisetzung kann z.B. durch Träger-gebundene Vorformen von Duftstoffen erfolgen. Eine Träger-gebundene Vorform eines Duftstoffes wird auch als "Pro-Fragrance" oder Duftspeicherstoff bezeichnet. In diesem Zusammenhang offenbart das US-Patent 6,949,680 die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird.

WO 2009/118219 A1 offenbart photoaktivierbare Substanzen, welche eine Freisetzung von cyclischen Terpenen oder cyclischen Terpenoiden ermöglichen.

WO 2015/124671 offenbart die Verwendung bestimmter Ketone als photoaktivierbare Substanzen, die in Gegenwart von Licht in einer photochemischen Fragmentierung einen Aktivstoff freisetzen. Der genannte Aktivstoff besitzt beispielsweise eine duftgebende Aktivität, die durch den photochemisch induzierten Zerfall erst verzögert und über einen längeren Zeitraum hinweg auf einer bestimmten Oberfläche freigesetzt wird.

Aufgabe der vorliegenden Erfindung war die Bereitstellung photoaktivierbarer Substanzen als Duftspeicherstoffe, welche die verzögerte Freisetzung von Riechstoffketonen, insbesondere von Damasconen, erlauben.

Gelöst wurde diese Aufgabe durch eine Verbindung der folgenden Formeln (VIII) bis (XI):

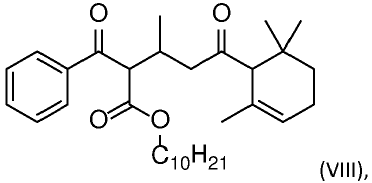

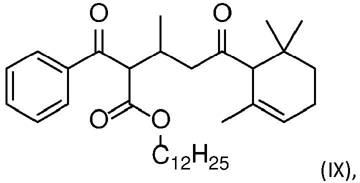

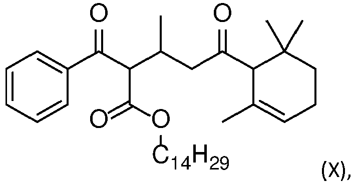

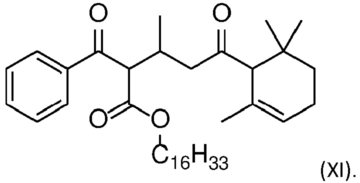

Es konnte überraschend gefunden werden, dass die erfindungsgemäßen Duftspeicherstoffe besonders wirksam sind, um die verzögerte Freisetzung von Riechstoffketonen, insbesondere von Damasconen, zu erlauben und dabei eine höhere Duftintensität bereitstellen, als die im Stand der Technik bekannten Photocages. Die Anwendung der erfindungsgemäßen Duftspeicherstoffe in Wasch-, Reinigungs- oder Pflegemitteln führt daher bei deren Anwendung zu einer verbesserten Langzeitduftwirkung, insbesondere im Zusammenhang mit der Textilbehandlung. Zum Beispiel konnte bei der Anwendung erfindungsgemäßer Duftspeicherstoffe in einem Wäschebehandlungsmittels, wie z.B. Waschmittel sowie Weichspüler, eine verbesserte Langzeitduftwirkung der behandelten Wäsche gefunden werden. Ferner weisen entsprechende Produkte eine besonders gute Lagerstabilität auf. Die erfindungsgemäßen Mittel ermöglichen es zudem, die Gesamtmenge an Parfüm, welches im Mittel enthalten ist, zu reduzieren, und dennoch Geruchsvorteile auf den gewaschenen Textilien zu erzielen, insbesondere mit Blick auf das Frischeempfinden.

Die erfindungsgemäßen Duftspeicherstoffe sind als Duftspeicherstoffe für alpha-Damascon.

Die vorstehend genannten Duftstoffe können als Rest R an die Verbindung der Formeln (VIII)-(XI) gebunden sein. Durch Einwirkung von Licht umfassend die Wellenlängen von 200 bis 600 nm können die gespeicherten Ketone freigesetzt werden.

"Substituiert", wie hierin in Bezug auf Alkyl-, Alkenyl-, Alkoxy- und Acylgruppen verwendet, bedeutet, dass die entsprechende Gruppe einen oder mehrere Substituenten aufweist, die ein oder mehrere Wasserstoffatome ersetzen, und ausgewählt werden aus -OR', -NR'R", -SR',-C(O)R', -C(O)OR', - C(O)NR'R", -NR-C(O)-R", und Halogen, wobei R' und R" für Wasserstoff oder unsubstituiertes C1-10 Alkyl stehen.

"Substituiert", wie hierin in Bezug auf Cycloalkyl-, Aryl- oder andere cyclische Kohlenwasserstoffreste verwendet, bedeutet, dass die entsprechende Gruppe einen oder mehrere Substituenten aufweist, die ein oder mehrere Wasserstoffatome ersetzen, und ausgewählt werden aus -OR', -NR'R", -SR',-C(O)R', -C(O)OR', -C(O)NR'R", -NR-C(O)-R", Halogen, und C1-10 Alkyl, =CR'R", oder C2-10 Alkenyl, wobei R' und R" für Wasserstoff oder unsubstituiertes C1-10 Alkyl stehen.

Erfindungsgemäß sind Duftspeicherstoffe entsprechend den nachfolgenden Formeln (VIII) bis (XI) geeignet.

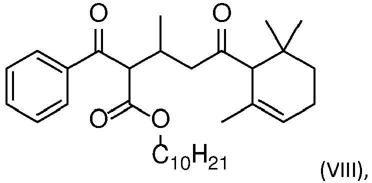

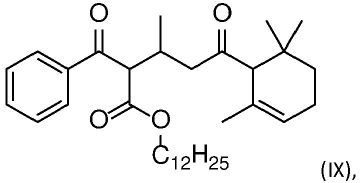

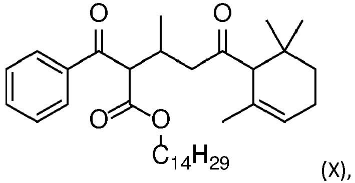

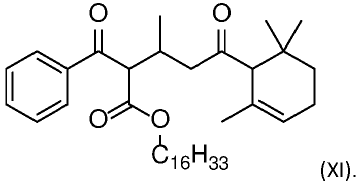

Die erfindungsgemäßen Duftspeicherstoffe lassen sich stabil in die üblichen Wasch- oder Reinigungsmittelmatrices, in Kosmetika und bestehende Riechstoffkompositionen einarbeiten. Sie ermöglichen eine verzögerte Freisetzung der gespeicherten Duftstoffe, unter anderem von Damascon in der alpha-, beta-, gamma- oder delta-Form sowie von Damascenon, insbesondere delta-Damascenon. Diese Duftspeicherstoffe verleihen üblichen Wasch- oder Reinigungsmitteln sowie Kosmetika einen besonders lange anhaltenden Frischeeindruck. Insbesondere das getrocknete, gewaschene Textil profitiert von der guten Langzeitfrischeduftwirkung. Die langsame Freisetzung des gespeicherten Riechstoffes erfolgt nach Einwirkung von Licht (elektromagnetische Strahlung) umfassend die Wellenlängen von 200 bis 600 nm, wie in der nachfolgenden, exemplarischen Reaktionsgleichung vereinfacht veranschaulicht, wobei die untenstehende Beispielverbindung nicht erfindungsgemäß ist, die Reaktionsgleichung aber analog für die erfindungsgemäßen Verbindungen gilt:

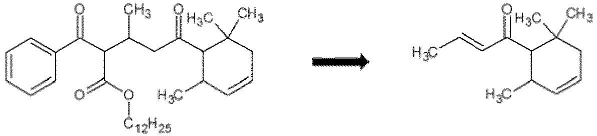

Ein weiterer Gegenstand der vorliegenden Erfindung ist ein Wasch- oder Reinigungsmittel, vorzugsweise ein Waschmittel, Weichspüler oder Waschhilfsmittel, enthaltend mindestens ein Duftspeicherstoff gemäß der Erfindung, wobei der Duftspeicherstoff vorzugsweise in einer Gesamtmenge zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist. Geeignete Reinigungsmittel sind z.B. Reinigungsmittel für harte Oberflächen, wie vorzugsweise Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken.

Gemäß einer bevorzugten Ausführungsform der Erfindung enthält das erfindungsgemäße Wasch- oder Reinigungsmittel mindestens ein Tensid, ausgewählt aus anionischen, kationischen, nichtionischen, zwitterionischen, amphoteren Tensiden oder Mischungen daraus.

Gemäß einer weiteren bevorzugten Ausführungsform der Erfindung liegt das erfindungsgemäße Mittel in fester oder flüssiger Form vor.

Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Ein weiterer Gegenstand der Erfindung ist ein kosmetisches Mittel, enthaltend mindestens ein Duftspeicherstoff gemäß der Erfindung, welches den Duftspeicherstoff vorzugsweise in einer Gesamtmenge zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthält.

Ein weiterer Gegenstand der Erfindung ist ein Luftpflegemittel (z.B. Raumlufterfrischer, Raumdeodorant, Raumspray usw.), enthaltend mindestens ein erfindungsgemäßen Duftspeicherstoff, wobei der genannte Duftspeicherstoff vorzugsweise in einer Gesamtmenge zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

Nach einer weiteren bevorzugten Ausführungsform der Erfindung sind in einem erfindungsgemäßen Mittel (d.h. Wasch- oder Reinigungsmittel, kosmetisches Mittel, oder Luftpflegemittel) zusätzliche Duftstoffe und/oder Duftspeicherstoffe enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limettenöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Fichtenöl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methylbeta-naphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylakohol, n-Octylaldehyd, p-Oxy-Acetophenon, Pentadekanolid, beta-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimatalkohol, Zimtsäure, Zimtsäureethylester, Zimtsäurebenzylester. Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Mittel (d.h. Wasch- oder Reinigungsmittel, kosmetisches Mittel, oder Luftpflegemittel), wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Einlaufverhinderer, Elektrolyte, Enzyme, Farbschutzstoffe, Färbemittel, Farbstoffe, Farbübertragungsinhibitoren, Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, Soil release-Wirkstoffe, UV-Schutz-Substanzen, Viskositätsregulatoren, Verdickungsmittel, Verfärbungsinhibitoren, Vergrauungsinhibitoren, Vitamine und/oder Weichspüler.

Die Mengen der einzelnen Inhaltsstoffe in den erfindungsgemäßen Mitteln (d.h. Wasch- oder Reinigungsmittel, kosmetisches Mittel oder Luftpflegemittel) orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen. Üblicherweise kann z.B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während z.B. Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäßen Mittel (d.h. Wasch- oder Reinigungsmittel, kosmetisches Mittel oder Luftpflegemittel) können Tenside enthalten, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar. Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X- für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammoniumbromid, Dioctyl-dimethyl-ammoniumchlorid, 1-Cetylpyridiniumchlorid und Thiazoliniodid sowie deren Mischungen. Bevorzugte QAV sind die Benzalkoniumchloride mit C₈-C₂₂-Alkylresten, insbesondere C₁₂-C₁₄-Alkylbenzyl-dimethylammoniumchlorid.

Bevorzugte Esterquats sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Handelsübliche Beispiele sind die von der Firma Stepan unter dem Warenzeichen Stepantex® vertriebenen Methylhydroxyalkyldialkoyloxyalkylammoniummethosulfate oder die unter dem Handelsnamen Dehyquart® bekannten Produkte der Firma BASF SE beziehungsweise die unter der Bezeichnung Rewoquat® bekannten Produkte des Herstellers Evonik.

Tenside sind in den erfindungsgemäßen Mitteln (d.h. Wasch- oder Reinigungsmittel, kosmetisches Mittel, oder Luftpflegemittel) in Mengenanteilen von vorzugsweise 5 Gew.-% bis 50 Gew.-%, insbesondere von 8 Gew.-% bis 30 Gew.-%, enthalten. Insbesondere in Wäsche-Nachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Mitteln insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, in den erfindungsgemäßen Mitteln vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wässriger Lösungen, die 3 Gew.-% bis 10 Gew.-% Wasserstoffperoxid enthalten, ggf. eingesetzt. Falls ein erfindungsgemäßes Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxo-hexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind, falls gewünscht, in den erfindungsgemäßen Mitteln vorzugsweise in Mengen nicht über 5 Gew.-%, insbesondere von 0,2 Gew.-% bis 2 Gew.-%, enthalten.

Die Mittel können ggf. als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Mittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt werden.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Apfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind optional in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel (d.h. insbesondere Wasch- oder Reinigungsmittel) bereitet keine Schwierigkeiten und kann im Prinzip in bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Die Herstellung der erfindungsgemäßen Duftspeicherstoffe wird im Beispielteil exemplarisch anhand der Herstellung von zwei delta-Damascone-enthaltenden Duftspeicherstoffen beschrieben. Über diese prinzipiellen Syntheserouten sind auch die erfindungsgemäßen Duftspeicherstoffe darstellbar.

Gemäß einer bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können.

Ein bevorzugtes erfindungsgemäßes festes, insbesondere pulverförmiges Waschmittel kann neben dem erfindungsgemäßen Duftspeicherstoff insbesondere noch Komponenten enthalten, die z.B. ausgewählt sind aus den folgenden:
- Aniontenside, wie vorzugsweise Alkylbenzolsulfonat, Alkylsulfat, z.B. in Mengen von 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-%, vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Duftstoffe
- ggf. Wasser
- ggf. Seife
- ggf. Bleichaktivatoren
- ggf. Cellulosederivate
- ggf. Schmutzabweiser,
Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Wasch- oder Reinigungsmittel sowie Kosmetika haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben dem erfindungsgemäßen Duftspeicherstoff insbesondere noch Komponenten enthalten, die z.B. ausgewählt sind aus den folgenden:
- Aniontenside, wie vorzugsweise Alkylbenzolsulfonat, Alkylsulfat, z.B. in Mengen von 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugter erfindungsgemäßer flüssiger Weichspüler kann neben dem erfindungsgemäßen Duftspeicherstoff insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Duftstoffe
- Farbstoffe, vorzugsweise im ppm-Bereich
- Stabilisatoren, vorzugsweise im ppm-Bereich
- Lösemittel, wie z.B. Wasser, in Mengen von vorzugsweise 60-90 Gew.-%,
- Gew.-% jeweils bezogen auf das gesamte Mittel.

Ein weiterer Gegenstand der Erfindung ist ein kosmetisches Mittel, wobei das kosmetische Mittel einen erfindungsgemäßen Duftspeicherstoff enthält.

Ein weiterer Gegenstand der Erfindung ist ein Verfahren zur lang anhaltenden Beduftung von Oberflächen, wobei ein erfindungsgemäßer Duftspeicherstoff oder ein erfindungsgemäßes Wasch- oder Reinigungsmittel auf die zu beduftende Oberfläche (z.B. Textil, Geschirr, Fußboden) aufgebracht wird und die genannte Oberfläche anschließend einer elektromagnetischen Strahlung umfassend die Wellenlängen von 200 bis 600 nm ausgesetzt wird.

Ein weiterer Gegenstand der Erfindung ist ein Verfahren zur lang anhaltenden Raumbeduftung, wobei ein erfindungsgemäßes Luftpflegemittel einer elektromagnetischen Strahlung umfassend die Wellenlängen von 200 bis 600 nm ausgesetzt wird.

### Beispiele

### Beispiel 1: Synthese von Dodecyl-2-benzoyl-3-methyl-5-oxo-5-(2,6,6-trimethylcyclohex-3-enyl)pentanoat

### Stufe 1: Benzoylessigsäuredodecylester

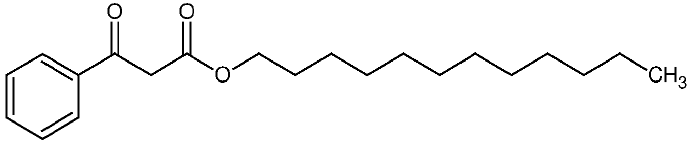

In einem Rundkolben wurden 10 mmol Benzoylessigsäureethylester, 12 mmol 1-Dodecanol in 30 ml Toluol vorgelegt und 6 h unter Rückfluss erhitzt. Das Lösungsmittel wurde abdestilliert, das entstandene Produkt wurde säulenchromatographisch aufgereinigt (EtOAc:c-Hex 1:10, R_{f} = 0.51); Ausbeute 97%.

### Stufe 2: Dodecyl-2-benzoyl-3-methyl-5-oxo-5-(2,6,6-trimethylcyclohex-3-enyl)pentanoat

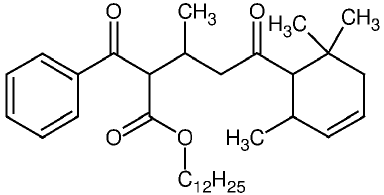

In einem Rundkolben wurde ein Gemisch aus 9 mmol Benzoylessigsäuredodecylester, 9 mmol delta-Damascon und 0,9 mmol Eisen(III)-Chlorid Hexahydrat in 2 ml Chloroform 48 h bei 50 °C gerührt. Anschließend wurde das Rohprodukt säulenchromatographisch aufgereinigt (MTBE:c-Hex, 1:5 R_{f} = 0.68); Ausbeute 88%.

### Beispiel 2: Freisetzungsverhalten

Die Testsubstanz wurde mol-gleich in Bezug auf den darin enthaltenen Riechstoff (entsprechend 0,4 Gew.-% delta-Damascon) in einen Weichspüler einformuliert, dieser wurde im Spülgang eines Waschprozesses verwendet. Die so behandelte Wäsche wurde nach dem Trocknen 1 min lang mit Hilfe des Sonnenlichtsimulationsgerät Atlas Suntest XXL+ mit 0,6 W/cm² bestrahlt. Die Läppchen befanden sich dabei in einer Petrischale mit Quarzdeckel (durchlässig für das gesamte Lichtspektrum). Nach der Bestrahlung wurde die Intensität des Duftes von 10 geruchlich geschulten Personen abgerochen, wobei jede Probe in zwei voneinander unabhängigen Durchgängen bewertet wurde, und mit Werten auf einer Skala von 1 bis 10 (10 = intensiv, 0 = kein Geruch) bewertet. Untersucht wurden der reine Riechstoff delta-Damascon als Referenz, die Verbindung aus Beispiel 1 und AB-32 (Verbindung aus dem Stand der Technik).

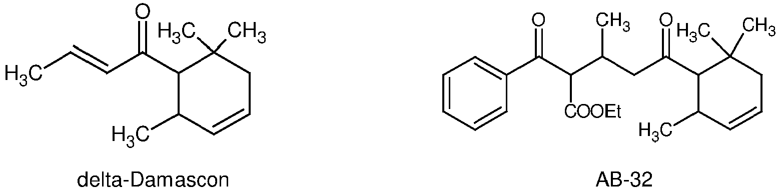

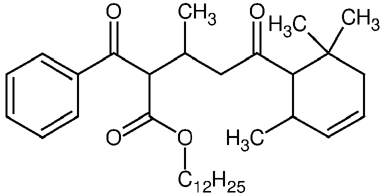

### Dodecyl-2-benzoyl-3-methyl-5-oxo-5-(2,6,6-trimethylcyclohex-3-enyl)pentanoat

| Probe | Duftintensität nach Bestrahlung |
|---|---|
| Referenz | 3 |
| Substanz aus Bsp. 1 | 6 |
| AB-32 | 4 |

Es zeigt sich, dass die obige Verbindung den bekannten Substanzen im Hinblick auf die Duftintensität überlegen ist.

## Patentansprüche

1. Verbindung, entsprechend einer der folgenden Formeln (VIII) bis (XI):

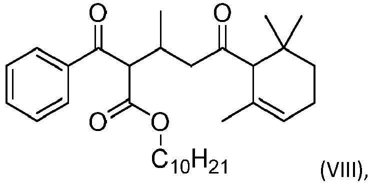

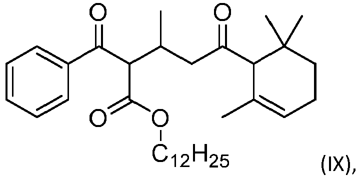

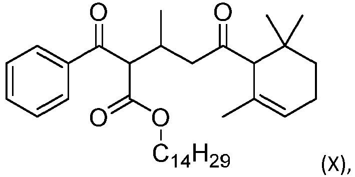

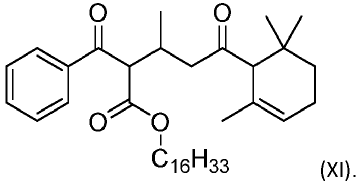

2. Wasch- oder Reinigungsmittel, enthaltend mindestens eine Verbindung nach Anspruch 1, wobei die genannte Verbindung vorzugsweise in einer Gesamtmenge zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

3. Wasch- oder Reinigungsmittel nach Anspruch 2, **dadurch gekennzeichnet, dass**
(1) es mindestens ein Tensid ausgewählt aus anionischen, kationischen, nichtionischen, zwitterionischen, amphoteren Tensiden oder Mischungen daraus enthält; und/oder
(2) es in fester oder flüssiger Form vorliegt.

4. Luftpflegemittel, enthaltend mindestens eine Verbindung nach Anspruch 1, wobei die genannte Verbindung vorzugsweise in einer Gesamtmenge zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

5. Kosmetisches Mittel, enthaltend mindestens eine Verbindung nach Anspruch 1, wobei die genannte Verbindung vorzugsweise in einer Gesamtmenge zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

6. Verfahren zur lang anhaltenden Beduftung von Oberflächen, **dadurch gekennzeichnet, dass** eine Verbindung nach Anspruch 1 oder ein Mittel nach Anspruch 2 oder 3 auf die zu beduftende Oberfläche aufgebracht wird und die genannte Oberfläche anschließend einer elektromagnetischen Strahlung umfassend die Wellenlängen von 200 bis 600 nm ausgesetzt wird.

7. Verfahren zur lang anhaltenden Raumbeduftung, **dadurch gekennzeichnet, dass** ein Luftpflegemittel nach Anspruch 4 enthalten ist, das einer elektromagnetischen Strahlung umfassend die Wellenlängen von 200 bis 600 nm ausgesetzt wird.

## Claims

1. A compound corresponding to one of the following formulas (VIII) to (XI):

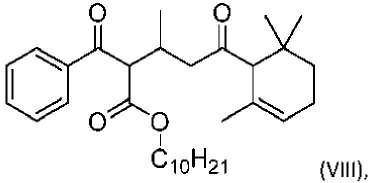

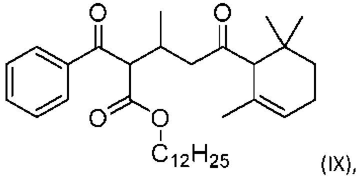

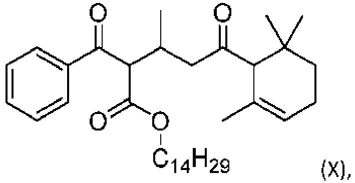

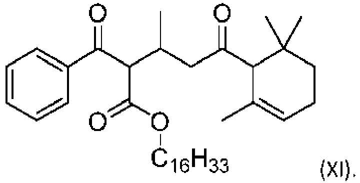

2. A washing or cleaning agent containing at least one compound according to claim 1, wherein said compound is preferably contained in a total amount of between 0.0001 and 5 wt.%, advantageously between 0.001 and 4 wt.%, more advantageously between 0.01 and 3 wt.%, in particular between 0.1 and 2 wt.%, in each case based on the total agent.

3. The washing or cleaning agent according to claim 2, **characterized in that**
(1) said washing or cleaning agent contains at least one surfactant selected from anionic, cationic, non-ionic, zwitterionic and amphoteric surfactants or mixtures thereof; and/or
(2) said washing or cleaning agent is present in solid or liquid form.

4. An air care agent containing at least one compound according to claim 1, wherein said compound is preferably contained in a total amount of between 0.0001 and 50 wt.%, advantageously between 0.001 and 5 wt.%, more advantageously between 0.01 and 3 wt.%, in particular between 0.1 and 2 wt.%, in each case based on the total agent.

5. A cosmetic agent containing at least one compound according to claim 1, wherein said compound is preferably contained in a total amount of between 0.0001 and 5 wt.%, advantageously between 0.001 and 4 wt.%, more advantageously between 0.01 and 3 wt.%, in particular between 0.1 and 2 wt.%, in each case based on the total agent.

6. A method for long-lasting fragrancing of surfaces, **characterized in that** a compound according to claim 1 or an agent according to claim 2 or 3 is applied to the surface to be fragranced and said surface is then exposed to electromagnetic radiation having wavelengths of from 200 to 600 nm.

7. A method for long-lasting fragrancing of a room, **characterized in that** an air care agent according to claim 4 is contained which is exposed to electromagnetic radiation having wavelengths of from 200 to 600 nm.

## Revendications

1. Composé selon l'une des formules (VIII) à (XI) suivantes :

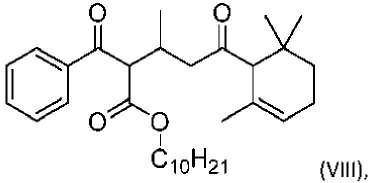

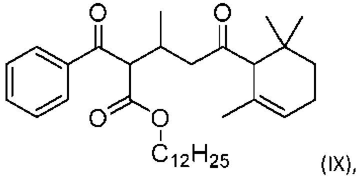

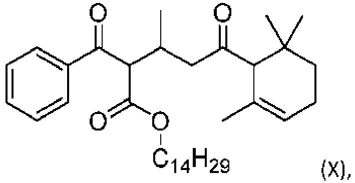

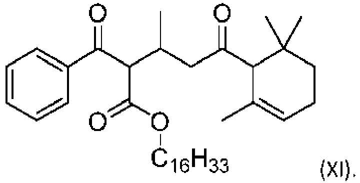

2. Agent de lavage ou de nettoyage contenant au moins un composé selon la revendication 1, dans lequel ledit composé est de préférence contenu en une quantité totale comprise entre 0,0001 et 5 % en poids, plus avantageusement entre 0,001 et 4% en poids, plus avantageusement encore entre 0,01 et 3 % en poids, en particulier entre 0,1 et 2 % en poids, dans chaque cas par rapport à la totalité de l'agent.

3. Agent de lavage ou de nettoyage selon la revendication 2, **caractérisé en ce**
(1) **qu'**il contient au moins un tensioactif choisi parmi les tensioactifs anioniques, cationiques, non ioniques, zwitterioniques, amphotères ou des mélanges de ceux-ci ; et/ou
(2) **qu'**il est sous forme solide ou liquide.

4. Agent de climatisation contenant au moins un composé selon la revendication 1, dans lequel ledit composé est de préférence contenu en une quantité totale comprise entre 0,0001 et 50 % en poids, plus avantageusement entre 0,001 et 5 % en poids, plus avantageusement encore entre 0,01 et 3 % en poids, en particulier entre 0,1 et 2 % en poids, dans chaque cas par rapport à la totalité de l'agent.

5. Agent cosmétique contenant au moins un composé selon la revendication 1, dans lequel ledit composé est de préférence contenu en une quantité totale comprise entre 0,0001 et 5 % en poids, plus avantageusement entre 0,001 et 4 % en poids, plus avantageusement encore entre 0,01 et 3 % en poids, en particulier entre 0,1 et 2 % en poids, dans chaque cas par rapport à la totalité de l'agent.

6. Procédé de parfumage durable des surfaces, **caractérisé en ce qu'**un composé selon la revendication 1 ou un agent selon la revendication 2 ou 3 est appliqué sur la surface à parfumer et ladite surface est ensuite exposée à un rayonnement électromagnétique comprenant des longueurs d'onde de 200 à 600 nm.

7. Procédé de parfumage durable d'une chambre, **caractérisé en ce qu'**un agent de soin de l'air selon la revendication 4 est contenu, lequel est exposé à un rayonnement électromagnétique comprenant des longueurs d'onde de 200 à 600 nm.
